Europäisches Patentamt

European Patent Office

Office européen des brevets

(11) Veröffentlichungsnummer: **0 414 128 A1**

# EUROPÄISCHE PATENTANMELDUNG

(21) Anmeldenummer: 90115688.5

(22) Anmeldetag: 16.08.90

(51) Int. Cl.⁵: **A61K 7/22**

(30) Priorität: 25.08.89 DE 3928063

(43) Veröffentlichungstag der Anmeldung:
27.02.91 Patentblatt 91/09

(84) Benannte Vertragsstaaten:
GR

(71) Anmelder: Henkel Kommanditgesellschaft auf
Aktien
Henkelstrasse 67
D-4000 Düsseldorf 13(DE)

(72) Erfinder: Wülknitz, Peter, Dr.
Am Alten Broich 69
D-4018 Langennfeld(DE)
Erfinder: Lehmann, Rudolf, Dr.
Schnugsheide 2
D-5653 Leichlingen 1(DE)
Erfinder: Plöger, Walter, Dr.
Holbeinweg 11
D-4010 Hilden(DE)
Erfinder: Förg, Franz, Dr.
Rotdornweg 10
D-4018 Langenfeld(DE)

(54) Belaghemmende Zahnpaste.

(57) Zahnpasten, die 10 - 60 Gew.-% Poliermittel, 2 - 20 Gew.-% Feuchthaltemittel, 0,5 - 5 Gew.-% wasserlösliche Konsistenzregler, 0,02 - 0,5 Gew.-% antibakterielle Biguanide und 1 - 5 Gew.-% weiterer Zusatzstoffe aus der Gruppe der oberflächenaktiven Stoffe, der Aromaöle und Süßungsmittel enthalten, weisen eine besonders hohe Biguanid-Verfügbarkeit auf, wenn als Poliermittel überwiegend α-Aluminiumoxid-trihydrat, als Konsistenzregler nichtionische Polysaccharidderivate, als oberflächenaktive Stoffe ein Alkylglykosid und gegebenenfalls ein nichtionogener Lösungsvermittler für das Aromaöl und als Süßungsmittel L-Aspartyl-L-phenylalanin-methylester enthalten sind.

## BELAGHEMMENDE ZAHNPASTE

Die Erfindung betrifft eine Zahnpaste in Form einer Dispersion wasserunlöslicher Poliermittel in einem wäßrigen Träger, die als plaquehemmende Komponente eine antimikrobiell wirksame Biguanidverbindung enthält, und deren übrige Komponenten nach Art und Menge so ausgewählt sind, daß trotz relativ niedriger Dosierung der antimikrobiell wirksamen Biguanidverbindungen eine optimale Hemmung der Zahnbelagsbildung erreicht wird.

Es ist seit langem bekannt, daß antimikrobiell wirksame Biguanidverbindungen eine die Bildung der Zahnbeläge verhindernde Wirkung haben. Diese Wirkung wird jedoch durch viele in Zahnpasten übliche Komponenten, insbesondere durch bestimmte Poliermittel, durch viele Bindemittel bzw. Konsistenzregler, oberflächenaktive Stoffe, ja sogar durch Süßungsmittel stark verringert oder ganz aufgehoben.

Es hat daher nicht an Versuchen gefehlt, solche Komponenten zu finden, welche die Wirkung der antimikrobiellen Biguanide nicht beeinträchtigen. So wird z. B. in DE-OS 21 58 149 vorgeschlagen, als Poliermittel $\alpha$-Aluminiumoxid-trihydrat in einer bestimmten Partikelgröße einzusetzen. In DE-OS 34 44 958 wird andererseits offenbart, daß bestimmte Tenside die Wirkung eines antimikrobiellen Biguanids verstärken können. Es wurde nun festgestellt, daß auch dieser Effekt durch bestimmte Zahnpastenkomponenten, z. B. durch anionische Bindemittel bzw. Konsistenzregler, anionische Süßstoffe und bestimmte Lösungsvermittler, die zur Solubilisierung gegebenenfalls enthaltener Aromaöle erforderlich sind, stark verringert wird. Eine Zahnpaste, die auch noch bei niedriger Dosierung der antimikrobiell wirksamen Biguanidverbindungen eine hohe Biguanid-Verfügbarkeit und eine befriedigende Plaque-Hemmung aufweist, ist dem Stand der Technik nicht zu entnehmen. Es war Ziel der vorliegenden Erfindung, eine solche Zahnpaste zu schaffen.

Gegenstand der Erfindung ist eine Zahnpaste in Form einer Dispersion, enthaltend

10 - 60 Gew.-% Poliermittel,

2 - 20 Gew.-% Feuchthaltemittel,

0,5 - 50 Gew.-% wasserlösliche Konsistenzregler,

0,02 - 0,5 Gew.-% antimikrobielle Biguanide,

1 - 5 Gew.-% weiterer Zusatzstoffe aus der Gruppe der oberflächenaktiven Stoffe, der Aromaöle und Süßungsmittel, dadurch gekennzeichnet, daß

- als Poliermittel überwiegend $\alpha$-Aluminiumoxid-trihydrat,

- als Konsistenzregler nichtionische Polysaccharidderivate,

- als oberflächenaktive Stoffe ein Alkylglykosid und gegebenenfalls ein nichtionogener Lösungsvermittler für das Aromaöl und

- als Süßungsmittel L-Aspartyl-L-phenylalanin-methylester enthalten sind.

$\alpha$-Aluminiumoxid-trihydrat, $Al(OH)_3$, ist ein bekanntes Poliermittel für Zahnpasten. Bevorzugt eignet sich eine gemahlene Qualität, deren Teilchen überwiegend (mindestens 98 kleiner als 50 $\mu$, mit einem Mittelwert von circa 1 - 10 $\mu$ sind.

Bevorzugt ist in der erfindungsgemäßen Zahnpaste eine Mischung aus $\alpha$-Aluminiumoxid-trihydrat (A) und einer schwach calcinierten Tonerde (B) im Gewichtsverhältnis A : B = 100 : (1 - 15) in einer Menge von 30 - 60 Gew.-% enthalten, weil dadurch eine besonders hohe Polierwirkung ohne aufrauhende Nebenwirkung erzielt wird.

Die schwach calcinierte Tonerde hat bevorzugt einen Gehalt von circa 20 Gew.-% Gamma-Aluminiumoxid ($\gamma$-$Al_2$-$O_3$) und circa 80 Gew.-% Alpha-Aluminiumoxid ($\alpha$-$Al_2O_3$), eine Agglomeratgröße unter 20 $\mu$, eine mittlere Primärkristallgröße von 0,5 - 1,5 $\mu$ und ein Schüttgewicht von 500 - 600 g/l.

Geeignete schwach calcinierte Tonerden werden durch Calcination aus Aluminiumhydroxid hergestellt. Aluminiumhydroxid geht durch Calcination in das bei Temperaturen oberhalb 1 200 $^\circ$C thermodynamisch stabile $\alpha$.$Al_2O_3$ über. Die bei Temperaturen zwischen 400 und 1 000 $^\circ$C auftretenden, thermodynamisch instabilen $Al_2O_3$-Formen bezeichnet man als Gamma-Formen (vgl. Ullmann, Encyclopädie der technischen Chemie, 4. Auflage (1974) Band 7, Seite 298). Durch Wahl der Temperatur und der Zeitdauer bei der Calcination kann man den Calcinationsgrad, d. h. die Umwandlung in das thermodynamisch stabile $\alpha$-$Al_2O_3$ auf beliebige Höhe einstellen. Man erhält durch schwache Calcination eine Tonerde mit einem Gehalt an $\gamma Al_2O_3$, der um so niedriger ist, je höher die Calcinationstemperatur und je länger die Calcinationsdauer gewählt wird. Schwach calcinierte Tonerden unterscheiden sich von reinem $\alpha$-$Al_2O_3$ durch eine geringere Härte der Agglomerate, eine größere spezifische Oberfläche und größere Porenvolumina.

Aluminiumoxid-Poliermittel verschiedener Calcinationsgrade, Mahlfeinheit und Schüttgewichte sind im Handel erhältlich, z. B. die "Poliertonerden" der Firma Giulini-Chemie.

Als Feuchthaltemittel kommen Glycerin, Sorbit, Propylenglykol und Polyethylenglykole in Frage, bevorzugt werden Glycerin und/oder Sorbit verwendet.

EP 0 414 128 A1

Geeignete wasserlösliche Konsistenzregler sind die nichtionischen Polysaccharidderivate, z. B. Methyl-, Hydroxyethyl- und Hydroxypropylether von Cellulose, Stärke, Guar und Pflanzengummen. Bevorzugt werden Hydroxyethylcellulose und Methylhydroxypropylcellulose eingesetzt.

Als antimikrobiell wirksame Biguanidverbindung wird bevorzugt das aus GB-A-705 838 bekannte 1,1'-Hexamethylenbis-[5-(4-chlorphenyl)biguanid] ("Chlorhexidin") in Form eines wasserlöslichen, physiologisch verträglichen Salzes, z. B. in Form des Acetats oder als Gluconat eingesetzt. Andere erfindungsgemäß geeignete antimikrobielle Biguanidverbindungen sind z. B. das 1,1'-Hexymethylen-bis-[5-(4-fluorphenyl)-biguanid] (Fluorhexidin) die aus GB-A-702 268 bekannten Polyhexamethylenbiguanidverbindungen des Typs Vantocil[R] IB (ICI), ferner die aus US-A-2 684 924, US-A-2 990 425, US-A-3 468 898, US-A-4 022 834, US-A-4 053 636 und US-A-4 198 392 bekannten antimikrobiellen Biguanidverbindungen.

Als oberflächenaktive Stoffe werden in den erfindungsgemäßen Zahnpasten nichtionogene Tenside vom Typ der Alkylglykoside eingesetzt. Alkylglykoside, ihre Herstellung und ihre Verwendung als oberflächenaktive Stoffe sind beispielsweise aus US-A-3 839 318, US-A-3 707 535, US-A-3 547 828, DE-A-19 43 689, DE-A-20 36 472 und DE-A-30 01 064 sowie EP-A-77 167 bekannt. Ihre Herstellung erfolgt insbesondere durch Umsetzung von Glucose oder Oligosacchariden mit primären Alkoholen mit 8 - 16 C-Atomen. Bezüglich des Glykosidrestes gilt, daß sowohl Monoglykoside, bei denen ein cyclischer Zuckerrest glykosidisch an den Fettalkohol gebunden ist, als auch oligomere Glykoside mit einem Olygomerisationsgrad (OG) bis vorzugsweise 3 geeignet sind. Bevorzugt geeignete Alkylglykoside für die Herstellung der erfindungsgemäßen Zubereitungen sind solche mit 8 - 18 C-Atomen in der Alkylgruppe und einem mittleren Oligomerisierungsgrad des Glycosidrestes von 1 - 3. Der Oligomerisierungsgrad (OG) ist dabei ein statistischer Mittelwert, dem eine für solche technischen Produkte übliche Homologenverteilung zugrunde liegt. Diese Alkylglykoside sind in der erfindungsgemäßen Zahnpaste bevorzugt in Mengen von 0,025 - 2,5 Gew.-% enthalten.

Die erfindungsgemäße Zahnpaste kann durch Zugabe von Aromaölen und Süßungsmitteln in ihren organoleptischen Eigenschaften verbessert werden. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z. B. Menthol, Carvon , Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Zur Solubilisierung dieser meist wasserunlöslichen Aromaöle in der Zahnpaste ist erfindungsgemäß ein nichtionogener Lösungsvermittler erforderlich. Solche Lösungsvermittler gehören zur Gruppe der oberflächenaktiven Verbindungen. Ein weiterer Gegenstand der Erfindung ist daher eine erfindungsgemäße Zahnpaste, die 0,1 - 0,5 Gew.-% eines Aromaöls und 0,1 - 0,7 Gew.-% eines nichtionogenen Lösungsvermittlers, bevorzugt aus der Gruppe der oxethylierten Fettsäureglyceride, der oxethylierten Fettsäuresorbitanpartialester oder Fettsäurepartialester von Glycerin- oder Sorbitan-Oxethylaten enthält. Lösungsvermittler aus der Gruppe der oxethylierten Fettsäureglyceride umfassen vor allem Anlagerungsprodukte von 20 - 60 Mol Ethylenoxid an Mono- und Diglyceride von linearen Fettsäuren mit 12 - 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 - 60 Mol Ethylenoxid an Sorbitanmonoester und Sorbitandiester von Fettsäuren mit 12 - 18 C-Atomen. Ebenfalls geeignete Lösungsvermittler sind Fettsäurepartialester von Glycerin- oder Sorbitan-Oxethylaten; das sind bevorzugt Mono- und Diester von $C_{12}$-$C_{18}$-Fettsäuren und Anlagerungsprodukten von 20 - 60 Mol Ethylenoxid an 1 Mol Glycerin oder an 1 Mol Sorbit.

Die erfindungsgemäße Zahnpaste enthält bevorzugt als Lösungsvermittler für gegebenenfalls enthaltene Aromaöle Anlagerungsprodukte von 20 - 60 Mol Ethylenoxid an gehärtetes oder ungehärtetes Ricinusöl (d. h. an Oxystearinsäure- oder Ricinolsäure-triglycerid), an Glycerin-mono- und/oder -distearat oder an Sorbitanmono-und/oder -distearat.

Als Süßungsmittel eignen sich entweder natürliche Zucker, z. B. Sucrose, Maltose, Lactose und Fructose oder synthetische Süßstoffe, jedoch bevorzugt nichtionogene oder amphotere Stoffe. Bevorzugt geeignet als Süßungsmittel ist der L-Aspartyl-L-phenylalanin-methylester, im Handel unter der Warenbezeichnung Aspartame[R].

Es können auch weitere bekannte Zahnpastenzusätze in untergeordneten Mengen von insgesamt bis zu höchstens 3 Gew.-% zugegeben werden, soweit diese mit dem antimikrobiellen Biguanid verträglich sind und dessen Wirkung nicht beeinträchtigen. Solche Zusätze sind z. B.

3

- karieshemmende Stoffe wie Natriumfluorid oder Natriummonofluorphospat,
- Pigmente wie z. B. Titandioxid,
- Farbstoffe
- pH-Stellmittel zur Einstellung eines pH-Wertes von bevorzugt 6 - 8 und Puffersubstanzen, z. B. Citronensäure und deren Salze oder Phosphorsäure und deren Alkalisalze,
- wundheilende und entzündungshemmende Stoffe wie z. B. Allantoin, Harnstoff, Azulen bzw. Kamillenwirkstoffe.

Die folgenden Beispiele sollen den Gegenstand der Erfindung näher erläutern ohne ihn hierauf zu beschränken.

| Beispiele | | | | | |
|---|---|---|---|---|---|
| Zahnpaste | | | | | |
| (Zusammensetzung in Gew.-%) | 1 | 2 | 3 | 4 | 5 |
| Aluminiumoxid-trihydrat[1] | 45,0 | 45,0 | 35,0 | 35,0 | 54,0 |
| Tonerde, schwach calciniert[2] | - | 1,0 | - | 1,0 | 1,0 |
| Glycerin | 10,0 | 10,0 | 15,0 | 15,0 | 5,0 |
| Sorbit (70 %ig) | - | - | - | - | 5,0 |
| Hydroxyethylcellulose[3] | 1,5 | 1,5 | 2,0 | 2,0 | 3,0 |
| Methylhydroxypropylcellulose[4] | 1,5 | 1,5 | 1,5 | 1,5 | - |
| Chlorhexidin-digluconat | 0,3 | 0,3 | 0,2 | 0,2 | 0,2 |
| Alkyl($C_{12/14}$)-glucosid (0 G = 1,3) | 0,25 | 0,25 | 0,25 | 0,25 | 0,5 |
| HR 60[5] | 0,4 | 0,4 | 0,1 | 0,1 | 0,2 |
| Pfefferminzöl | 0,4 | 0,4 | 0,1 | 0,1 | 0,2 |
| Aspartame(R) | 0,2 | 0,2 | 0,1 | 0,1 | 0,1 |
| Wasser | ad 100 | ad 100 | ad 100 | ad 100 | ad 100 |

[1] Es wurde Hydrated Alumina, Grade C 333 der Fa. ALCOA Chem. Div. eingesetzt
[2] Es wurde das Handelsprodukt Poliertonerde eingesetzt
[3] Es wurde das Handelsprodukt Cellobond HEC 400 T von BP eingestzt
[4] Es wurde das Handelsprodukt Culminal MHPC 100 von Henkel eingesetzt
[5] Anlagerungsprodukt von 60 Mol Ethylenoxid an gehärtetes Ricinusöl

**Ansprüche**

1. Zahnpaste, in Form einer wäßrigen Dispersion, enthaltend
10 - 60 Gew.-% Poliermittel,
2 - 20 Gew.-% Feuchthaltemittel,
0,5 - 5 Gew.-% wasserlösliche Konsistenzregler,
0,02 - 0,5 Gew.-% antimikrobielle Biguanide und
1 - 5 Gew.-% weiterer Zusatzstoffe aus der Gruppe der oberflächenaktiven Stoffe, der Aromaöle und Süßungsmittel, dadurch gekennzeichnet, daß
- als Poliermittel überwiegend α-Aluminiumoxid-trihydrat,
- als Konsistenzregler nichtionische Polysaccharidderivate,
- als oberflächenaktive Stoffe ein Alkylglykosid und gegebenenfalls ein nichtionogener Lösungsvermittler für das Aromaöl und
- als Süßungsmittel L-Aspartyl-L-phenylalanin-methylester enthalten sind.
2. Zahnpaste nach Patentanspruch 1, dadurch gekennzeichnet, daß als Poliermittel eine Mischung aus α-Aluminiumoxid-trihydrat (A) und einer schwach calcinierten Tonerde (B) im Gewichtsverhältnis A : B = 100 : (1 - 15) in einer Menge von 30 - 60 Gew.-% enthalten ist.
3. Zahnpaste nach Patentanspruch 1 oder 2, dadurch gekennzeichnet, daß als antimikrobielles Biguanid das 1,1'-Hexamethylen-bis-(4-chlorphenyl)-biguanid (Chlorhexidin) in Form eines wasserlöslichen Salzes enthalten ist.

4

4. Zahnpaste nach einem der Patentansprüche 1 - 3, dadurch gekennzeichnet, daß als Alkylglykoside solche mit 8 - 18 C-Atomen in der Alkylgruppe und einem mittleren Oligomerisationsgrad des Glycosidrestes von 1 - 3 in einer Menge von 0,025 bis 2,5 Gew.-% enthalten ist.

5. Zahnpaste nach einem der Patentansprüche 1 - 4, dadurch gekennzeichnet, daß 0,1 - 0,5 Gew.-% eines Aromaöls und 0,1 - 0,7 Gew.-% eines Lösungsvermittlers aus der Gruppe der oxethylierten Fettsäureglyceride, der oxethylierten Fettsäuresorbitanpartialester oder der Fettsäurepartialester von Glycerin-oder Sorbitan-Oxethylaten enthalten ist.

**Europäisches Patentamt**

# EUROPÄISCHER RECHERCHENBERICHT

Nummer der Anmeldung

**EP 90 11 5688**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DE-A-2 126 539   (COLGATE-PALMOLIVE)<br>* Seite 11, Zeilen 15-19; Seite 19, Beispiel 3 *<br>– – – | 1-5 | A 61 K 7/22 |
| Y | EP-A-0 026 252   (BLENDAX)<br>* Beispiel 1 *<br>– – – | 1-5 | |
| Y | EP-A-0 304 627   (HENKEL)<br>* Patentansprüche; Beispiele 1,2; Seite 3, Zeilen 40-42 *<br>– – – – – | 1-5 | |

| RECHERCHIERTE SACHGEBIETE (Int. Cl.5) |
|---|
| A 61 K |

Der vorliegende Recherchenbericht wurde für alle Patentansprüche erstellt

| Recherchenort | Abschlußdatum der Recherche | Prüfer |
|---|---|---|
| Den Haag | 21 November 90 | WILLEKENS G.E.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument